# EUROPEAN PATENT SPECIFICATION

(11) **EP 3 897 813 B1**
(45) Date of publication and mention of the grant of the patent: **11.09.2024**
(21) Application number: 19831804.0
(22) Date of filing: 17.12.2019
(51) Int. Cl.: A61M 39/22, A61F 5/44, A61F 5/445

(54) **A VALVE FOR A UROSTOMY APPLIANCE**
VENTIL FÜR EIN UROSTOMIEGERÄT
VALVE POUR APPAREIL D'UROSTOMIE

(30) Priority: 18.12.2018 GB 201820596; 28.02.2019 GB 201902714
(43) Date of publication of application: 27.10.2021
(73) Proprietor: Salts Healthcare Limited, West Midlands B7 4AA (GB)
(72) Inventor: ALLEN, Marcus, Birmingham West Midlands B7 4AA (GB)
(74) Representative: Forresters IP LLP
(86) International application number: PCT/GB2019/053593
(87) International publication number: WO 2020/128456

(56) References cited:
- WO-A1-2016/187350
- US-A1- 2002 000 253

## Description

The present invention relates to a valve for a urostomy appliance.

Urostomy appliances are well known in the field. They are typically attached to a patient by an adhesive wafer which extends around the patient's stoma with adhesive and provide a collecting chamber to collect waste exiting the stoma. A mechanism for draining the collecting chamber is often provided - typically, these are in the form of a tap or bung, which allow the patient to open an outlet from the urostomy appliance and drain the contents, for example, into a toilet.

US2002/0000253 discloses a valve apparatus that includes a housing having proximal and distal ends and defining a passageway between. An access port of the housing defines a bore configured for selective communication with the passageway. WO2016/187350 discloses a spring-loaded bag connector having a spring-loaded valve and a housing comprising a fluid inlet portion and a fluid outlet portion.

In accordance with an aspect of the present invention we provide a valve for a urostomy appliance including: a housing for connection to the urostomy appliance, the housing having an inlet and an outlet, wherein the housing includes a housing passage extending between the inlet and outlet, and a body which is received in the housing passage and which is rotatable about an axis between an open position, in which liquid is permitted to flow to the outlet, and a closed position, in which liquid is substantially prevented from flowing to the outlet, wherein the body is received in the opening in the housing and has a body passage extending from a body inlet to a body outlet, wherein when the body is in its open position the body outlet at least partially aligns with the housing outlet and permits liquid to pass through the body passage and flow through the housing outlet, and wherein the valve includes a sealing device which extends around a periphery of the body between the body and the housing passage and which extends around the body outlet, such that the sealing device prevents ingress of liquid between the body and the housing passage when the body is in its closed position.

Further aspects of the present invention are provided in the appended claims 2 to 9.

Embodiments of the invention will now be described with reference to the accompanying figures, of which:
Figure 1 shows a urostomy appliance;
Figure 2 shows a side view of the urostomy appliance of Figure 1;
Figure 3 shows a perspective view of an embodiment of a valve;
Figure 4 shows another perspective view of an embodiment of the valve;
Figure 5 shows another perspective view of an embodiment of the valve;
Figure 6 shows an exploded view of an embodiment of the valve;
Figure 7A and 7B show the internal configuration of an embodiment of a valve in a closed and open position;
Figure 8 shows an exploded view of a body of an embodiment of a valve, and
Figure 9 shows an embodiment of the valve.

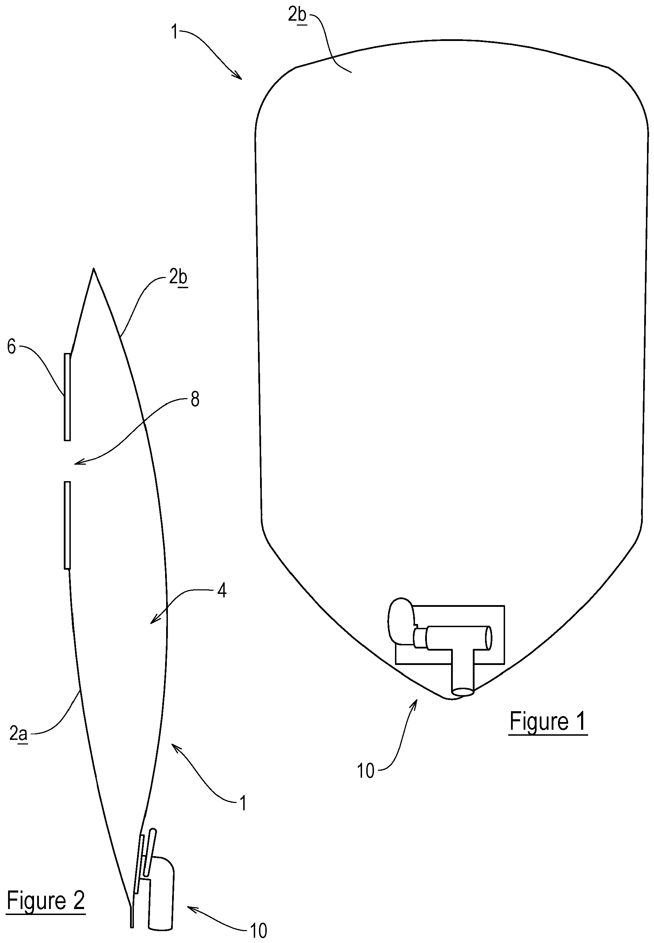

With reference to figures 1 and 2, particularly, a urostomy appliance 1 is illustrated. The urostomy appliance 1 includes first and second walls 2a, 2b which are connected together (for example, via a heat weld) to form a waste collecting cavity 4. The first wall 2a is attached to an adhesive wafer 6. An aperture (known as a stoma receiving opening 8) extends through both the first wall 2a and the adhesive wafer 6 to provide an entrance to the waste collecting cavity 4.

A valve 10 (described in more detail below) is attached to the second wall 2b of the urostomy appliance 1. The valve 10 communicates with the waste collecting cavity 4 and has an open and a closed position, in which waste is or is not permitted to flow through the valve 10 (i.e. flow out of the waste collecting cavity 4).

In use, the patient attaches the adhesive wafer 6 around their stoma. Waste liquid (for example, urine and/or blood and/or other body fluids) exits the body, via the stoma, and flows through the stoma receiving opening 8 and is collected in the waste collecting cavity 4. The valve 10 is selectively used to permit the waste which is collected in the waste collecting cavity 4 to flow out of the appliance 1, through the valve 10.

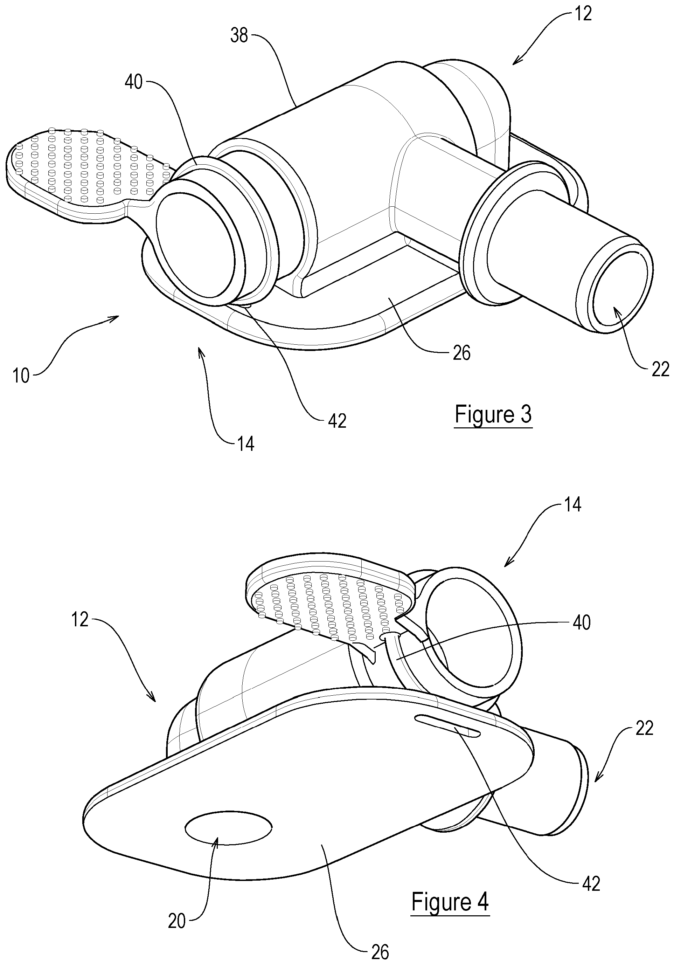

Features of the valve 10 are shown in more detail in figures 3 to 8. The valve 10 includes a housing 12 which connects to the urostomy appliance 1, and a body 14, which is supported in the housing 12. The housing 12 has an inlet 20, an outlet 22 and a housing passage 24. A liquid flow path extends between the inlet 20 and the outlet 22, through at least a part of the housing passage 24 (see flow path "B" illustrated in 7B, particularly).

In the illustrated embodiment (see especially figures 3 and 4), the housing 12 also includes a planar member 26 and a cylindrical portion 38. The planar member 26 forms the part of the housing 12 that connects to the second wall 2b. The inlet 20 is defined in the planar member 26. An aperture (not shown) in the second wall 2b corresponds with the inlet 20 of the housing 12 (which provides a path for waste between the waste collecting cavity 4 and the valve 10).

In the present example, the housing 12 attaches to the second wall 2b by a heat weld which seals the wall 2b to the planar member 26. It should be appreciated that the housing 12 may be attached to the urostomy appliance 1 by another means, so long as the housing 12 is sealed to the urostomy appliance 1, so that liquid cannot exit the appliance 1 (in normal use) without using the valve 10.

In this example, the cylindrical portion 38 defines the housing passage 24. However, it should be appreciated that this exact configuration is not essential.

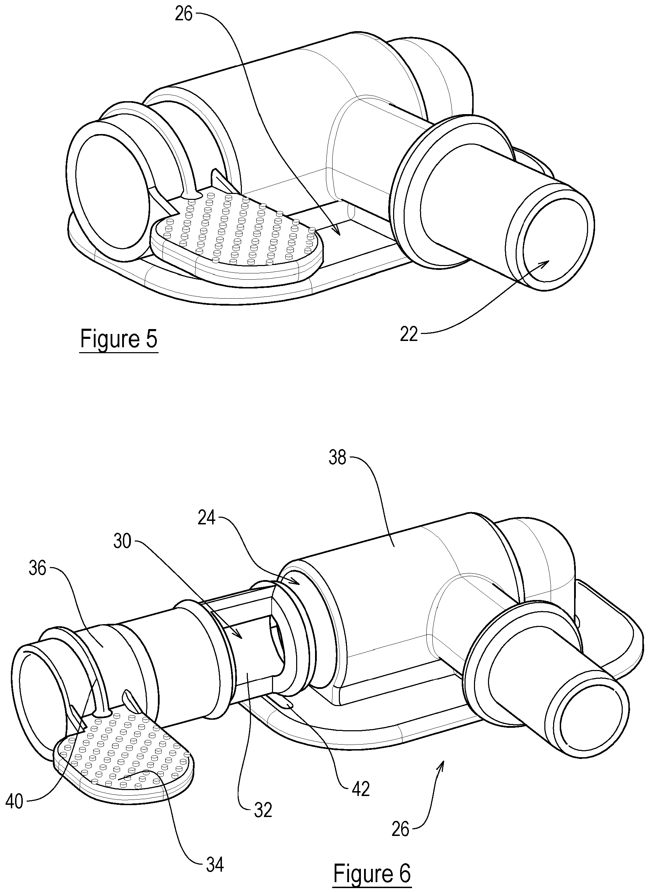

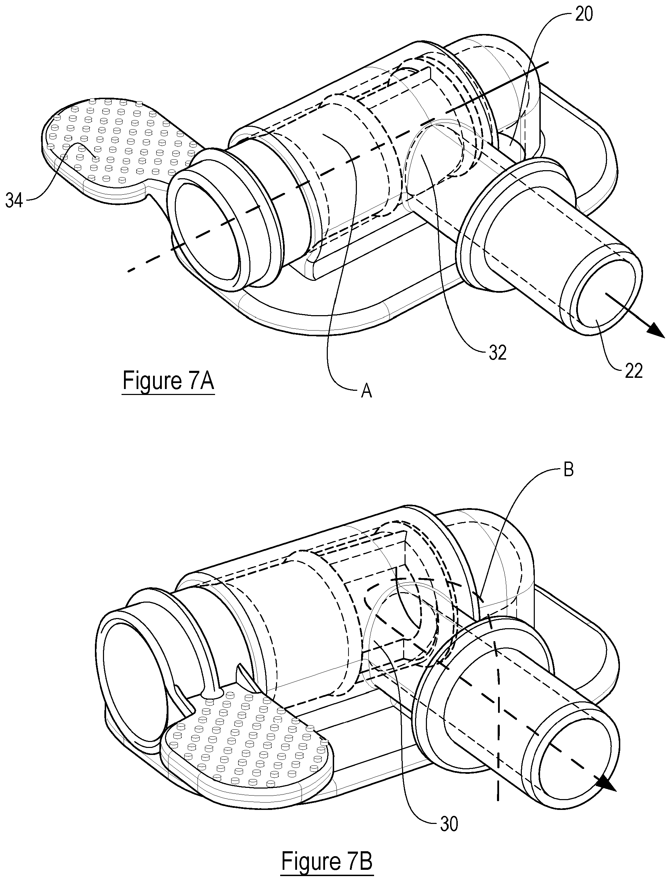

The body 14 is received in the housing passage 24 and is rotatable between an open position (shown in figures 5 and 7B) and a closed position (shown in figures 3 and 7A). In the open position, liquid can flow through the valve 10. In this example, this includes the waste liquid flowing out of a waste collecting cavity 4 via the aperture in the second wall 2b and the inlet 20 of the valve 10 and out of the outlet 22. In the closed position, liquid is prevented from flowing through the valve 10 (therefore, the waste is stored in the waste collecting cavity 4).

The body 14 has a blocking portion 32 and defines a body inlet and a body outlet 30 (and a body passage extending from the body inlet to the body outlet 30). In the illustrated example, the body 14 has a wall which forms a substantially cylindrical part 36 and the body outlet 30 is defined through the wall of the cylindrical part 36.

The body 14 also has a user operable portion 34 for effecting rotation of the body. The cylindrical part 36 is received in the cylindrical portion 38 / housing passage 24 of the housing 12.

In more detail, in the open position, the body outlet 30 at least partially aligns with the outlet 22 and permits liquid to flow through the outlet 22. In the closed position, the blocking portion 32 aligns with the outlet 22 and liquid is substantially prevented / inhibited from exiting through the outlet 22. In other words, the body outlet 30 opens the flow path through the valve 10 and the blocking portion 32 closes the flow path through the valve 10, and the position of the blocking portion 32 and body outlet 30 depends on the rotational position of the body 14 relative to the housing 12.

The body 14 rotates between its open and closed position about an axis of rotation (illustrated by the line referenced "A" in the figure 7A). The axis "A" extends substantially centrally through the body 14 and the housing passage 24 (i.e. the cylindrical portion 38 of the housing 12 that defines the housing passage 24).

The valve 10 also includes an engagement device which prevents or at least inhibits the body 14 from being removed from the passage 24 during rotation of the body 14. The engagement device includes a first and a second formation 40, 42. The first formation 40 is provided on or by the body 14 and extends, at least partially, around the body. The second formation 42 is located outside an entrance to the passage 24 and engages with the first formation 40 (when the valve 10 is assembled).

The first formation 40 is positioned in a plane which is substantially transverse to the axis. In some embodiments, the first formation 40 extends at least half way around a circumference of the body 14 (and, in an example extends completely around the circumference of the body 14).

In the illustrated embodiment, the first formation 40 includes a ridge or a rim which projects from an outer surface of the body 14. As can be seen from the figures, the projection in this example extends continuously around the outer surface of the body 14. It should be appreciated that the projection / first formation 40 need not extend continuously and such an embodiment is described further below.

The second formation 42 extends transverse to the axis of rotation of the body 14. The second formation 42 is substantially elongate and, in the present example, includes a recess/groove or an aperture.

The second formation 42 (in this example, an elongate aperture) is formed in the planar member 26 of the housing 12 (adjacent to the entrance to the passage 24). The first formation 40 (in the illustrated example, a projection) is received in the second formation when the body 14 is in an operating position in the passage of the housing 12 (i.e. in the configuration in which liquid is inhibited from exiting through the valve 10 when the valve 10 is closed).

In some embodiments, the first formation 40 may include multiple projections which are spaced apart from each other around the outer surface of the body 14. In order to ensure the engagement device fulfils its function of inhibiting the body 14 being removed from the housing 12 when the body 14 is rotated, the projections are spaced at an appropriate distance so that as one projection exits an end of the recess or aperture of the second formation 42 (when the body 14 rotates), another projection enters the recess at an opposing end. In other words, at least one projection is always received in the recess at all times/at all body positions.

In some embodiments, the plane in which the first formation 40 is positioned also passes through user operable portion 34 (refer to the figures, for example). The first formation 40 may extend completely around the body 14 except for the peripheral space which is occupied by the user operable portion 34. In other words, the first formation 40 extends completely around the circumference of the body 14 and has a first end which is connected to one side of the user operable portion 34 and an opposite second end which is connected to a second side of the user operable portion 34.

The valve 10 is assembled by inserting the body 14 into the passage 24 of the housing 12. The body 14 is pushed into the housing 12 until the first formation 40 is positioned in / engages with the second formation 42 on the housing 12. Once the first formation 40 is held in the second formation 42, the valve 10 is in an "operational positon" - i.e. the body 14 may be rotated in the housing 12 between the above described open and closed position in order to permit or block liquid from exiting through the outlet 22 of the housing 12 and the engagement device prevents the body 14 becoming detached from the housing 12.

There are multiple features that may be provided alone or in combination with each other to aid assembly of the valve 10. In some embodiments, the planar member 26 includes a camming surface which leads to the second formation 42 and engages the first formation 40 as the body 14 is inserted into the passage 24. In other words, the camming surface aids assembly because the body 14 is subject to a gradually increasing force as the first formation 40 is moved along the camming surface. In some embodiments, the camming surface is positioned at or near a periphery of the planar member 26 (and between an edge of the planar member 26 and the second formation 42).

In some embodiments, the planar member 26 is made from a relatively more flexible material than other parts of the housing 12 / body 14. This allows a degree of "flexing" as the body 14 is moved into position (e.g. its operable position in which the first formation 40 engages the second formation 42). In this case, the planar member 26 flexes "away" from the first formation 40 as the body 14 is moved towards its position in the second formation 42. In other words, the planar member 26 becomes more convex on its top surface, temporarily, to aid movement of the body 14 (and the first formation 40) into position and then returns to its normal shape (i.e. a flatter shape) once the first formation 40 is received by the second formation 42 (and the body 14 is held in place relative to the housing 12).

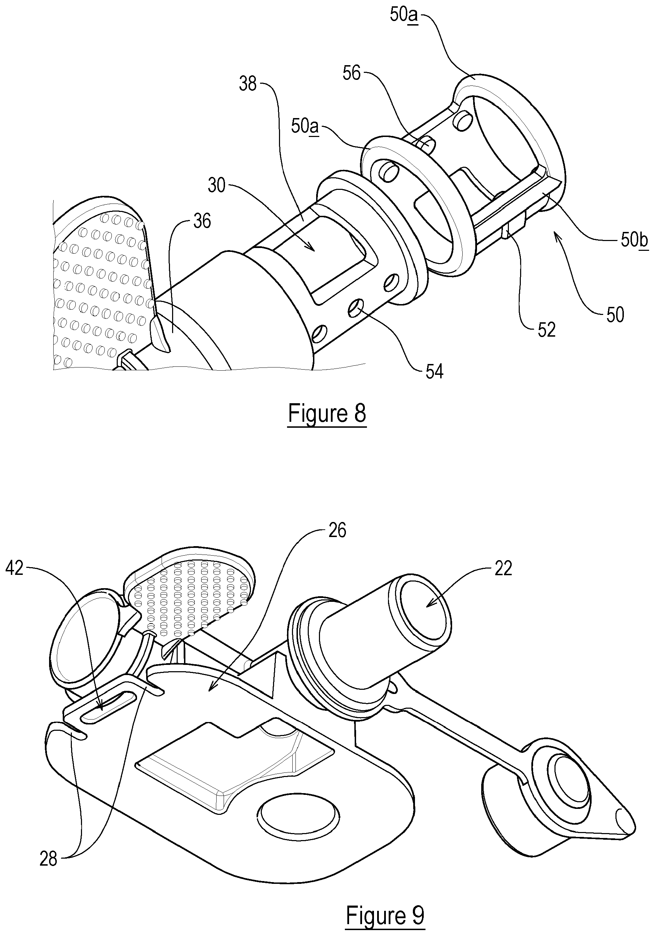

In the embodiment illustrated in figure 9, the valve 10 has the features that are described in relation to the other figures (some of which are labelled explicitly to aid the explanation included below). It should be appreciated, that any embodiment could include any combination of one or more optional features described.

In the case of the valve 10 in figure 9, the planar member 26 includes two slits 28, one of which is located on each opposing side of the second formation 42 (i.e. each slit 28 is positioned adjacent the second formation 42). In other words, part of the planar member 26 forms of three portions that are separated by elongate openings that extend inwards from the periphery of the planar member 26 - the second formation 42 is positioned on the central portion (of the three portions) between the two openings.

The slits 28 extend parallel to each other and extend inwardly from the periphery of the planer member 26 (although it should be appreciated that the slits could extend at an angle to one another and provide similar functionality). The slits 28 allow a portion of the planar member 26 to flex/move as the body 14 is moved into its operable position (where the first and second formations 40, 42 engage) relative to the remaining parts of the planar member 26. In other words, the portion of the planar member 26, between the slits 28, is able to deform out of position (and out of alignment with the rest of the planar member 26), so that moving the body 14 into operational position is easier. The planar member 26 moves back to its original position once the first and second formations 40, 42 engage. It should be appreciated that the slits 28 do not necessarily have to extend through all of the material of the planar member 26. Alternatively, a single or pair or more groove(s) could be provided that also permit the planar member to deflect / distort from its resting position and aids assembly of the valve 10.

It should be appreciated that, in some embodiments, there may be a single slit positioned in a location so as to allow a portion of the planar member 26 to distort as the body 14 is moved towards its operational position.

Additionally, it should be appreciated that the slit(s) described above may be combined with one or more of the other features described above. For example, a camming surface maybe provided on the planar member and/or the slit(s) and/or manufacturing from a relatively more flexible material in any combination, as desired.

The body 14 further includes a sealing device 50 which extends around a periphery of the body 14, between the body 14 and the housing passage 24, and the sealing device 50 also extends around the body outlet 30 so that the sealing device 50 prevents the ingress of liquid between the body 14 and the housing 12 when the body 14 is in the closed position. The wording "around" is intended to define the sealing device 50 extending in a plane generally transverse to the rotation axis.

In some embodiments (for example, those illustrated in the figures), the sealing device 50 is on the body 14. In other words, the body 14 supports the sealing device 50. This is advantageous because it allows the sealing device 50 to provide the required sealing around the body outlet 30 to prevent liquid ingress between the body 14 and the housing 12 (even when the body 14 is rotated to a different position).

The sealing device 50 includes two generally parallel annuli 50a which extend around the body 14 and are spaced apart from another on opposing axial sides of the body outlet 30. There are two further joining portions 50b which extend from one annulus 50a to the other, on either side of the body outlet 30. In this way two sealing rings around the periphery of the body 14 are provided and a seal surrounding the body outlet 30 also.

A surface of the sealing device 50 that faces outwards from the body 14 may be substantially convex or curved.

In some embodiments, the sealing device 50 includes two support portions 52 (each of which includes a relatively thicker portion of material). Each support portion 52 is positioned approximately mid-way between the parallel annuli 50a and connects to one of the joining portions 50b. Each support portion 52 helps reduce distortion of the sealing device 50. Over time, as the valve 10 is used and the body 14 is repeatedly rotated in the housing 12 a distorting force will be exerted on the edges of the sealing device 50 (particularly parts of the sealing device 50 that surround the body outlet 30) which may bend the sealing device 50 out of shape and/or out of position. In some circumstances, this could result in the sealing device 50 failing and allowing liquid through the valve 10 even when the body 14 is in its closed position. The support portions 52 are more rigid than other parts of the sealing device 50 (by virtue of their relative thickness) and, thus, reinforce the sealing device 50. It should be appreciated that the sealing device 50 may include more or fewer support portions 52, as desired.

In some embodiments, a retention device which aids retention of the sealing device 50 in a correct location with respect to the body 14 is present. The retention device includes, in this example, six recesses 54 which are generally circle shaped and formed in the body 14 and six corresponding projections 56 (in this case, the projections are also generally circle shaped) which project inwardly from an inner surface of the sealing device 50. When the sealing device 50 is in position on the body 14, the projections 56 engage the recesses 54. This provides anchorage to help keep the sealing device 50 in the correct position.

It should be appreciated that although there are six recesses 54 and six projections 56 in the illustrated example, this need not be the case. There may be, in some embodiments, one or more recesses, which engage the same number of projections and they may be differently shaped, e.g. square, oval or hexagonal. In other words, at least one recess and projection correspond to one another such that they can function to provide anchoring of the sealing device 50 in position relative to the body 14.

In some embodiments, the sealing device 50 is manufactured in position relative to the body 14 by using an overmoulding process. The sealing device 50 is made with a thermoplastic elastomer / thermoplastic rubber material (although other suitable materials could be used). It is important that the material is capable of distorting such that when it presses between the body 14 and the housing 12, it can provide the necessary sealing.

The housing 12 is made from a High Density Polyethylene (HOPE) resin. Such a material provides toughness, rigidity and strength to the housing 12. The body 14 is made from a polypropylene homopolymer (as such, the body 14 has low warpage and high stiffness in combination with moderate impact strength at room temperature).

In use, the waste collecting cavity 4 collects waste (e.g. urine and/or other body fluid) which enters the urostomy appliance 1 via the stoma opening 8. The valve 10 is attached to the second wall 2b of the urostomy appliance 1, so that the user can select when they wish to empty the waste collecting cavity 4.

While the valve 10 is in the closed position, the blocking portion 32 seals the flow path to the outlet 22 of the housing 12 (as illustrated in figure 7A). To open the valve, the user uses the user operable portion 34 to rotate the body 14 within the housing 12 (about axis "A").

To open / close the valve 10, the body 14 is rotated between first and second positions (the relative alignments of the outlets is discussed in detail below). In this example, the body 14 is held and/or maintained in a single axial position relative to the housing 12 (i.e. the body 14 does not enter the housing 12 further, nor does it move out of the housing 12 at all). In the illustrated example, the first formation 40 is held in a single axial position by the second formation 42. In other words, the first formation 40 is permitted to rotate in / through the second formation 42, so that the body 14 remains held in a single axial position (along axis "A") relative to the housing 12.

In the open position, the body 14 is in a positon in which the body outlet 30 aligns with the outlet 22 of the housing and, thus, liquid is permitted to flow out of the valve 10 (this is shown in figure 7B). The flow path "B" created when the valve 10 is in the open position is illustrated in figure 7B.

In the closed position (illustrated in figure 7A), the body 14 is in a position in which the body outlet 30 is out of alignment with the outlet 22 of the housing 12. In this example, the wall of the housing 12 provides the blocking portion 32 which blocks the housing outlet 22 and prevents or at least inhibits liquid flow out of the body outlet 30 (and the housing outlet 22). In other words, while the body outlet 30 is out of alignment with the housing outlet 22, the wall of the body 14 is in alignment with the housing outlet 22, so as to block the flow of fluid through the valve 10.

In summary, the body 14 is rotated between open and closed positions and, in the open position the body outlet 30 aligns with the housing outlet 22 (and the blocking portion 32 is out of alignment with the housing outlet 22), and in the closed position the body outlet 30 is out of alignment with the housing outlet 22 (and the blocking portion 32 is in alignment with the housing outlet 22).

In the illustrated embodiment, the distance between the valve 10 being in a fully closed position (i.e. where the blocking portion 32 is centred across an opening which leads to the outlet 22 and "completely" blocking the liquid flow) and the fully open position (i.e. where the body outlet 30 aligns with the opening which leads to the outlet 22 and the flow path is "completely" open) is a half turn or around 180 degrees. It should be appreciated that the open position may not be the fully open position as the flow path may still be open when the body 14 has completed a smaller rotation (e.g. around 90 degrees).

However, the maximum flow rate of liquid from the urostomy appliance 1 will be lower because the flow path is narrower.

The valve 10 is closed (and sealed to prevent or at least inhibit liquid exit from the valve 10) via a rotational movement of the body 14 relative to the housing 12, so as to bring the sealing device 50, into complete contact with an interior surface of the housing 12 (which blocks the liquid flow path between the body inlet and outlet 30 and the housing outlet 22). As discussed above, the sealing device 50 is located around the body outlet 30 and seals against the housing 12 to prevent (or at least inhibit) liquid flow.

The valve 10 is opened (which allows liquid to flow through the valve 10) via a rotational movement of the body 14 relative to the housing 12, so as to open the liquid flow path through the body outlet 30.

It should be appreciated that although the user operable portion 34 permits easier use for the user of the valve 10, it is not essential for the functioning of the valve 10 itself.

## Claims

1. A valve (10) for a urostomy appliance (1) including:
a housing (12) for connection to the urostomy appliance (1), the housing (12) having an inlet (20) and an outlet (22), wherein the housing (12) includes a housing passage (24) extending between the inlet (20) and outlet (22), and
a body (14) which is received in the housing passage (24) and which is rotatable about an axis between an open position, in which liquid is permitted to flow to the outlet (22), and a closed position, in which liquid is substantially prevented from flowing to the outlet (22), wherein the body (14) is received in the opening in the housing (12) and has a body passage extending from a body inlet to a body outlet (30),
wherein when the body (14) is in its open position the body outlet (30) at least partially aligns with the housing outlet (22) and permits liquid to pass through the body passage and flow through the housing outlet (22), and
wherein the valve (10) includes a sealing device (50) which extends around a periphery of the body (14) between the body and the housing passage (24) and which extends around the body outlet (30), such that the sealing device (50) prevents ingress of liquid between the body (14) and the housing passage (24) when the body (14) is in its closed position.

2. A valve according to claim 1 wherein the sealing device (50) includes a portion that extends around the body (14) and which lies in a plane substantially transverse to the rotation axis.

3. A valve according to any of the preceding claims wherein the sealing device (50) includes one or more support portions (52).

4. A valve according to any of the preceding claims wherein the sealing device (50) includes two substantially parallel annulus (50a) each of which extends around the periphery of the body (14) with each annulus (50a) positioned to a respective axial side of the body outlet (30).

5. A valve according to claim 4 wherein the annulus (50a) are connected to each other by a pair of joining portions (50b) which together with the annulus completely surround the body outlet (30).

6. A valve according to any of the preceding claims including a retention device which holds the sealing device (50) in a correct location.

7. A valve according to claim 6 wherein the retention device includes at least one recess (54) in the body (14) and at least one projection (56) on the sealing device or vice versa.

8. A valve according to any of the preceding claims wherein the sealing device is overmoulded on the body (14).

9. A urostomy appliance (1) including:
a first wall (2a) and a second wall (2b) connected substantially about their peripheries to define an internal waste collecting cavity (4),
a stoma receiving opening (8) which is positioned in the first wall (2a) and is in communication with the waste collecting cavity (4), and
a valve (10) according to any of claims 1-8.

## Patentansprüche

1. Ventil (10) für ein Urostomiegerät (1), umfassend:
Ein Gehäuse (12) zum Anschluss an das Urostomiegerät (1), wobei das Gehäuse (12) einen Einlass (20) und einen Auslass (22), wobei das Gehäuse (12) einen Gehäusedurchgang (24) einschließt, der sich zwischen dem Einlass (20) und dem Auslass (22) erstreckt, und
einen Körper (14), der im Gehäusedurchgang (24) aufgenommen wird und der um eine Achse zwischen einer offenen Position, in welcher Flüssigkeit zum Auslass (22) fließen kann, und einer geschlossenen Position, drehbar ist, in welcher Flüssigkeit wesentlich daran gehindert wird zum Auslass (22) zu fließen, wobei der Körper (14) in die Öffnung im Gehäuse (12) aufgenommen wird und einen Körperdurchgang aufweist, der sich von einem Körpereinlass zu einem Körperauslass (30) erstreckt,
wobei, wenn sich der Körper (14) in seiner offenen Position befindet, sich der Körperauslass (30) zumindest teilweise mit dem Gehäuseauslass (22) ausrichtet und Flüssigkeit durch den Körperdurchgang und durch den Gehäuseauslass (22) fließen lässt, und
wobei das Ventil (10) eine Dichtungsvorrichtung (50) einschließt, die sich um einen Umfang des Körpers (14) zwischen dem Körper und dem Gehäusedurchgang (24) erstreckt und sich um den Körperauslass (30) so erstreckt, dass die Dichtungsvorrichtung (50) Eindringen von Flüssigkeit zwischen dem Körper (14) und dem Gehäusedurchgang (24) verhindert, wenn sich der Körper (14) in seiner geschlossenen Position befindet.

2. Ventil nach Anspruch 1, wobei die Dichtungsvorrichtung (50) einen Abschnitt einschließt, der sich um den Körper (14) erstreckt und welcher in einer Ebene im Wesentlichen quer zur Drehachse liegt.

3. Ventil nach irgendeinem der vorhergehenden Ansprüche, wobei die Dichtungsvorrichtung (50) einen oder mehrere Stützabschnitte (52) einschließt.

4. Ventil nach irgendeinem der vorhergehenden Ansprüche, wobei die Dichtungsvorrichtung (50) zwei wesentlich parallele Ringe (50a) einschließt, sich jeder davon um den Umfang des Körpers (14) erstreckt, wobei jeder Ring (50a) an einer entsprechenden axialen Seite des Körperauslasses (30) positioniert ist.

5. Ventil nach Anspruch 4, wobei die Ringe (50a) durch ein Paar von Verbindungsabschnitten (50b) miteinander verbunden sind, die zusammen mit den Ringen den Körperauslass (30) völlig umgeben.

6. Ventil nach irgendeinem der vorhergehenden Ansprüche, das eine Haltevorrichtung einschließt, welche die Dichtungsvorrichtung (50) an der richtigen Stelle hält.

7. Ventil nach Anspruch 6, wobei die Haltevorrichtung zumindest eine Vertiefung (54) im Körper (14) und zumindest einen Vorsprung (56) an der Dichtungsvorrichtung oder umgekehrt einschließt.

8. Ventil ach irgendeinem der vorhergehenden Ansprüche, wobei die Dichtungsvorrichtung auf den Körper (14) geformt ist.

9. Urostomiegerät (1), das umfasst:
Eine erste Wand (2a) und eine zweite Wand (2b), die im Wesentlichen an ihren Ränder verbunden sind, einen inneren Abfallsammelhohlraum (4) zu definieren,
ein Stoma aufnehmende Öffnung (8), die in der ersten Wand (2a) positioniert und in Kommunikation mit dem Abfallsammelhohlraum (4) ist, und
ein Ventil (10) nach irgendeinem der Ansprüche 1-8.

## Revendications

1. Valve (10) pour un appareil d'urostomie (1) comprenant :
un boîtier (12) à connecter à l'appareil d'urostomie (1), le boîtier (12) comportant une entrée (20) et une sortie (22), dans lequel le boîtier (12) comprend un passage de boîtier (24) s'étendant entre l'entrée (20) et la sortie (22), et
un corps (14) qui vient se loger dans le passage de boîtier (24) et qui pout tourner autour d'un axe entre une position ouverte, dans laquelle du liquide peut s'écouler vers la sortie (22), et une position fermée, dans laquelle le liquide ne peut essentiellement s'écouler vers la sortie (22), dans lequel le corps (14) vient se loger dans l'ouverture à l'intérieur du boîtier (12) et comporte un passage de corps s'étendant d'une entrée de corps à une sortie de corps (30),
dans lequel, lorsque le corps (14) se trouve dans sa position ouverte, la sortie de corps (30) au moins en partie s'aligne sur la sortie de boîtier (22) et permet au liquide de passer à travers le passage de corps et de d'écouler à travers la sortie de boîtier (22), et
dans lequel la valve (10) comprend un dispositif d'étanchéité (50) qui s'étend sur un pourtour du corps (14), entre le corps et le passage de boîtier (24), et qui s'étend sur le pourtour de la sortie de corps (30), de telle sorte que le dispositif d'étanchéité (50) empêche l'entrée de liquide entre le corps (14) et le passage de boîtier (14) lorsque le corps (24) lorsque le corps (14) se trouve dans sa position fermée.

2. Valve selon la revendication 1, dans lequel le dispositif d'étanchéité (50) comprend une position qui s'étend autour du corps (14) et qui est située dans un plan sensiblement transversal par rapport à l'axe de rotation.

3. Valve selon l'une quelconque des revendications précédentes, dans lequel le dispositif d'étanchéité (50) comprend une ou plusieurs parties de support (52).

4. Valve selon l'une quelconque des revendications précédentes, dans lequel le dispositif d'étanchéité (50) comporte deux anneaux (50a) sensiblement parallèles, qui chacun s'étend sur le pourtour du corps (14), chaque anneau (50a) étant positionné par rapport à un côté axial respectif de la sortie de corps (30).

5. Valve selon la revendication 4, dans lequel les anneaux (50a) sont joints l'un à l'autre par une paire de parties de fixation (50b) qui, associées aux anneaux, entourent intégralement la sortie de corps (30).

6. Valve selon l'une quelconque des revendications précédentes comprenant un dispositif de retenue qui maintient le dispositif d'étanchéité (50) dans une position correcte.

7. Valve selon la revendication 6, dans lequel le dispositif de retenue comprend au moins un renfoncement (54) ménagé dans le corps (14) et au moins une saillie (56) sur le dispositif d'étanchéité ou vice versa.

8. Valve selon l'une quelconque des revendications précédentes, dans lequel le dispositif d'étanchéité est surmoulé sur le corps (14).

9. Appareil d'urostomie (1) comprenant :
une première paroi (2a) et une seconde paroi (2b) sensiblement jointes de part et d'autre des pourtours pour définir une cavité de collecte de déchets internes (4),
une ouverture de recueil de stomie (8) qui est située dans la première paroi (2a) et qui est en communication avec la cavité de recueil de déchets (4), et
une valve (10) selon l'une quelconque des revendications 1 à 8.
